# EUROPÄISCHE PATENTSCHRIFT

(11) **EP 3 166 504 B1**
(45) Veröffentlichungstag und Bekanntmachung des Hinweises auf die Patenterteilung: **02.11.2022**
(21) Anmeldenummer: 15729120.4
(22) Anmeldetag: 04.06.2015
(51) Int. Cl.: A61B 17/04

(54) **VORRICHTUNG ZUM KNÜPFEN EINES KNOTENS**
DEVICE FOR TYING A KNOT
DISPOSITIF POUR FAIRE UN NOEUD

(30) Priorität: 07.07.2014 DE 102014213135; 19.03.2015 DE 102015104153
(43) Veröffentlichungstag der Anmeldung: 17.05.2017
(73) Patentinhaber: Charité - Universitätsmedizin Berlin, 10117 Berlin (DE)
(72) Erfinder: FIKATAS, Panagiotis, 10439 Berlin (DE)
(74) Vertreter: Gulde & Partner
(86) Internationale Anmeldenummer: PCT/EP2015/062520
(87) Internationale Veröffentlichungsnummer: WO 2016/005118

(56) Entgegenhaltungen:
- EP-A2- 0 634 143
- EP-A2- 0 634 143
- WO-A2-2004/004577
- WO-A2-2004/004577
- WO-A2-2004/004577
- DE-T2- 69 818 737
- US-A- 5 144 961
- US-A- 5 144 961
- US-A- 5 217 470
- US-A- 5 405 352
- US-A- 5 405 352
- US-A- 5 454 821
- US-A- 5 643 293
- US-A- 5 643 293
- US-A- 5 681 331
- US-A- 5 681 331

## Beschreibung

Die Erfindung betrifft eine Vorrichtung Knüpfen eines Knotens.

Das Knüpfen bzw. Schließen von Knoten eines Fadens ist insbesondere bei beengten Platzverhältnissen schwierig und zeitaufwändig. Dies ist insbesondere der Fall, wenn besondere Anforderungen an die Festigkeit bzw. Haltbarkeit des Knotens gestellt werden. Derartige Anwendungen sind beispielsweise im Sport, beim Angeln, bei Verpackungsverfahren oder in der Medizintechnik zu finden.

Beispielhafte Ausführungen von Knoten des Standes der Technik sind offenbart in WO 2004/004577 A2, US 5,643,293 A, US 5,681,331 A, EP 0 634 143 A2, US 5,144,961 A sowie US 5,405,352 A.

Der Erfindung liegt nun die Aufgabe zugrunde, das Knüpfen eines Knotens zu verbessern. Diese Aufgabe wird gelöst mit einer Vorrichtung gemäß Anspruch 1. Vorteilhafte Ausgestaltungen finden sich in den abhängigen Ansprüchen.

Die erfindungsgemäße Vorrichtung zum Knüpfen eines Knotens mit einem Faden aufweisend ein Funktionsende und ein Zugende sieht vor, dass ein Grundkörper vorgesehen ist, um dessen Außenfläche der Faden in Form eines vorbereiteten, noch offenen Knotens gelegt ist, so dass durch Ziehen an dem Zugende der Faden an der Außenfläche entlanggleitend einen geschlossenen Knoten bildet.

Die erfindungsgemäße Vorrichtung hat den Vorteil, dass der Knoten bereits vor der Verwendung des Knotens vorbereitet wird, so dass bei der tatsächlichen Erstellung oder Verwendung des Knotens dieser nur noch zugezogen werden muss. Dies spart Zeit und erleichtert das Verknoten insbesondere bei schwer zugänglichen Anwendungen. Der mit dem Faden zu verknotende oder zu verbindende Gegenstand kann entweder durch eine Schlaufe des Fadens in dem Bereich des Funktionsendes geführt werden oder das Funktionsende kann mit dem Gegenstand in Kontakt gebracht werden, zum Beispiel umschlungen werden, und dann kann der Knoten zugezogen werden.

In bevorzugter Ausgestaltung der Erfindung ist vorgesehen, dass das Funktionsende (12) an einem Fixierungspunkt des Grundkörpers (2) befestigbar ist. Dies vereinfacht die Handhabung, das lediglich ein Fadenende betätigt werden muss.

In bevorzugter Ausgestaltung der Erfindung ist vorgesehen, dass der Fixierungspunkt an einer oberen oder unteren Deckfläche des Grundkörpers, an einer Innenfläche des Grundkörpers oder an der Außenfläche angeordnet ist. Der Fixierungspunkt kann an einer Außenfläche des Grundkörpers, aber auch im Inneren des Grundkörpers, zum Beispiel in einer Aussparung oder Ausnehmung, angeordnet sein. Je nach Einsatzzweck oder Form des Grundkörpers kann der Fixierungspunkt vorgesehen sein. Es können auch mehrere Fixierungspunkte vorgesehen sein, wobei der vorteilhafteste ausgewählt werden kann.

In weiterer bevorzugter Ausgestaltung der Erfindung ist vorgesehen, dass an dem Faden vor dem Funktionsende Befestigungsmittel zur Verbindung mit einem Gegenstand und/oder Funktionsmittel zur funktionalen Verbindung mit einem Gegenstand angeordnet sind. Die Befestigungsmittel und/oder Funktionsmittel sind vorzugsweise in räumlicher Nähe zu dem Funktionsende angeordnet und können fest oder verschiebbar beziehungsweise lösbar oder nicht lösbar an dem Faden angeordnet sein.

In bevorzugter Ausgestaltung der Erfindung ist vorgesehen, dass das Befestigungsmittel und/oder das Funktionsmittel eine Nadel, einen Anker und/oder eine künstliche Sehne umfasst. Die Befestigungsmittel können zum Beispiel Anker, Nadeln oder Haken umfassen. Die Funktionsmittel können funktionale Elemente wie zum Beispiel eine künstliche Sehne oder dergleichen umfassen.

In bevorzugter Ausgestaltung der Erfindung ist vorgesehen, dass an dem Fixierungspunkt flexibles Material angeordnet ist und dass an dem Funktionsende eine Spitze angeordnet ist, welche in das flexible Material stechbar ist. Auf diese Weise kann das Funktionsende einfach fixiert werden und zudem wird eine mögliche Verletzungsgefahr durch die Spitze, wie eine Nadel, verringert.

In weiterer bevorzugter Ausgestaltung der Erfindung ist vorgesehen, dass der Fixierungspunkt und das Funktionsende mittels einer Rastverbindung, einer Bajonettverbindung, einer Klebverbindung oder einer Schließverbindung verbindbar sind, wobei die Verbindung unlösbar oder wieder verschließbar sein kann. So kann das Funktionsende zum Beispiel per clip oder Haken anknüpfbar sein. Diese Verbindungen können entweder einmalig oder mehrmals geöffnet beziehungsweise geschlossen werden.

In weiterer bevorzugter Ausgestaltung der Erfindung ist vorgesehen, dass ein den Grundkörper und Faden umgebender Hüllkörper vorgesehen ist, wobei der Hüllkörper mindestens eine im Bereich der Mantelfläche angeordnete Öffnung für das Funktionsende und/oder das Zugende aufweist. Der Hüllkörper, der vorzugsweise eine etwas kürzere Erstreckung in Längs- oder axialer Richtung als der Grundkörper hat, schützt den gelegten Faden und vereinfacht die Handhabung.

Besonders vorteilhaft ist, dass der Grundkörper zylinderförmig, kegelstumpfförmig, quaderförmig, prismatisch oder speichenförmig mit radialen Ausläufern ausgebildet ist. Dann gleitet der Faden bei Zug an dem Zugende einfacher in Richtung des Funktionsendes beziehungsweise der Grundkörper kann optimal an den Faden, Knoten und/oder Einsatzzweck angepasst werden. Der Grundkörper kann massiv, mit Ausnehmungen versehen oder hohl, wie zum Beispiel ein Rohr, sein.

Der Faden weist einen dem Funktionsende zugewandten Adaptionsknoten und einen dem Zugende zugewandten Sicherungsknoten auf. Dies erlaubt eine modulare Anpassung des Knotens an die zu erfüllende Aufgabe. Über den Adaptionsknoten oder Adaptionsknotenbereich, der auch als Funktionsknoten oder Funktionsknotenbereich bezeichnet werden kann, werden die Eigenschaften des Knotens wie zum Beispiel Festigkeit, Zuziehverhalten, Größe etc. definiert. Der Sicherungsknoten oder Sicherungsknotenbereich dient dazu, den Adaptionsknoten gegen ein ungewolltes Auflösen oder Entknoten zu sichern.

Desweiteren ist vorgesehen, dass der Faden in einer ersten Richtung mindestens zweimal um den Grundkörper und in einer zweiten Richtung mindestens einmal um das Funktionsende gelegt ist. Die zweite Richtung ist entgegengesetzt zu der ersten Richtung. Beispielsweise verläuft die erste Richtung im Uhrzeigersinn und die zweite Richtung entgegen dem Uhrzeigersinn. Es hat sich gezeigt, dass ein derartiger Knoten relativ einfach vorzubereiten ist. So ist das Legen oder Vorbereiten auf dem Grundkörper relativ einfach und der benötigte Faden ist nicht sehr lang. Zudem ist der Knoten einfach zu schließen oder zu knüpfen und hält sicher.

In Ausgestaltung der Erfindung ist vorgesehen, dass der Faden zur Bildung eines Adaptionsknotens in einer ersten Richtung mindestens zweimal um den Grundkörper und in einer zweiten Richtung mindestens einmal um das Funktionsende gelegt ist und dass der Faden zur Bildung eines Sicherungsknotens in der zweiten Richtung mindestens einmal um den Grundkörper gelegt ist. Auch dieser Knoten ist einfach vorzubereiten und zu handhaben bei guter Festigkeit des zugezogenen Knotens.

In weiterer bevorzugter Ausgestaltung der Erfindung ist vorgesehen, dass an dem Funktionsende ein Element zur Befestigung an einem Gegenstand angeordnet ist. Das Element kann bereits von dem Faden durchdrungen und/oder umwickelt sein. Beispielsweise kann eine künstliche Sehne zur Befestigung an einem Gelenk bereits an dem Faden angeordnet sein. So kann ein vorbereitetes Kit für diese Anwendung angeboten werden, was den Aufwand bei einer Operation erheblich verringert.

Das offenbarte Verfahren (welches nicht ein Teil der Erfindung ist) zum Knüpfen eines Knotens mit einem Faden aufweisend ein Funktionsende und ein Zugende, ist gekennzeichnet durch die Schritte:
- Bereitstellen eines Grundkörpers, um dessen Außenfläche der Faden in Form eines vorbereiteten, noch offenen Knotens gelegt ist;
- Kontaktieren eines Gegenstands mit dem Funktionsende; und
- Ziehen an dem Zugende, so dass der Faden an der Außenfläche entlanggleitend einen geschlossenen Knoten bildet.

Es gelten die gleichen Vorteile und Modifikationen wie zuvor beschrieben.

In bevorzugter Ausgestaltung der Erfindung ist vorgesehen, dass das Funktionsende an einem Fixierungspunkt des Grundkörpers befestigt wird. Dies erleichtert die Handhabung, da nur ein Faden betätigt werden muss.

In bevorzugter Ausgestaltung der Erfindung ist vorgesehen, dass der Gegenstand mit dem Funktionsende umschlungen und/oder durchdrungen wird. Bei dieser Art des Kontakts mit dem Gegenstand wird das Funktionsende einmal oder mehrmals um den Gegenstand gelegt oder geführt, um den Gegenstand oder einen Teil oder Bereich des Gegenstands durch den Knoten zu fixieren. Für das Durchdringen kann das Funktionsende eine Spitze haben oder es kann eine Nadel an dem Funktionsende befestigt sein. Nachdem der Gegenstand einmal oder mehrmals durchdrungen ist, wird das Funktionsende an dem Grundkörper befestigt, woraufhin der Knoten zugezogen wird.

Die Erfindung wird nachfolgend in Ausführungsbeispielen anhand der zugehörigen Zeichnungen erläutert. Es zeigen:
Figur 1 eine perspektivische Darstellung einer Vorrichtung zum Knüpfen eines Knotens,
Figur 2 eine Draufsicht auf die Vorrichtung zum Knüpfen eines Knotens,
Figur 3 eine perspektivische Darstellung der Vorrichtung mit zu einem ersten Knoten gelegten Faden,
Figur 4 eine perspektivische Darstellung der Vorrichtung zum Knüpfen mit einem zu einem weiteren Knoten gelegten Faden,
Figur 5 eine perspektivische Darstellung der Vorrichtung zum Knüpfen mit einem zu einem weiteren Knoten gelegten Faden,
Figur 6 eine perspektivische Darstellung der Vorrichtung zum Knüpfen mit einem zu einem weiteren Knoten gelegten Faden,
Figur 7 eine perspektivische Darstellung der Vorrichtung zum Knüpfen mit einem zu einem weiteren Knoten gelegten Faden,
Figur 8 eine perspektivische Darstellung der Vorrichtung zum Knüpfen mit einem zu einem weiteren Knoten gelegten Faden,
Figur 9 eine perspektivische Darstellung der Vorrichtung zum Knüpfen mit einem zu einem weiteren Knoten gelegten Faden,
Figur 10 eine perspektivische Darstellung der Vorrichtung zum Knüpfen mit einem zu einem weiteren Knoten gelegten Faden,
Figur 11 eine perspektivische Darstellung einer weiteren Vorrichtung zum Knüpfen eines Knotens,
Figur 12 die weitere Vorrichtung zum Knüpfen eines Knotens mit einem weiteren Knoten und
Figur 13 die weitere Vorrichtung mit einem weiteren Knoten.

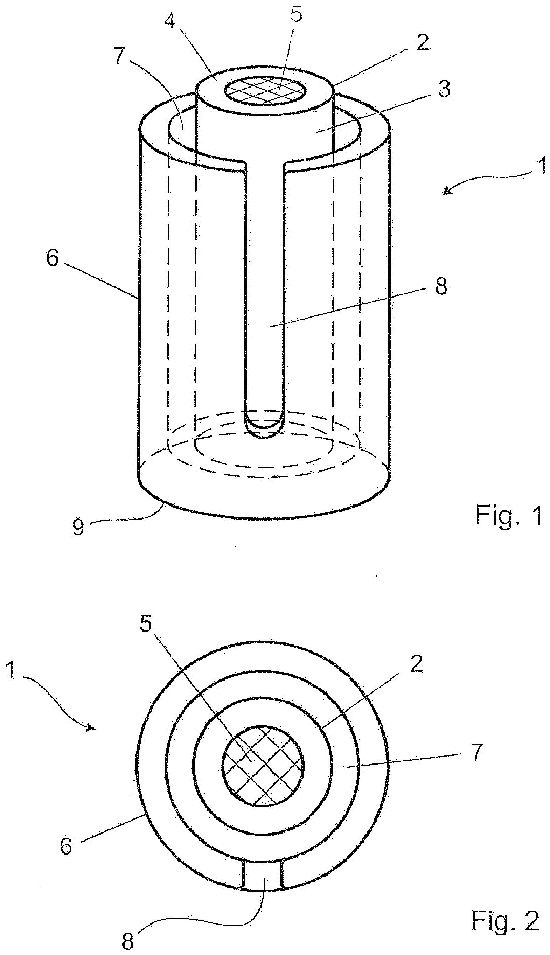

Figur 1 zeigt eine Vorrichtung 1 zum Knüpfen eines Knotens. Aus Gründen der Übersichtlichkeit ist zunächst die Vorrichtung 1 in den Figuren 1 und 2 ohne Faden dargestellt. Die Vorrichtung 1 umfasst einen in diesem Beispiel zylindrischen Grundkörper 2, der vorzugsweise kegelstumpfförmig ausgebildet ist. Der zylindrische Grundkörper 2 hat eine Mantelfläche 3 und eine Deckfläche 4. Auf oder an der Deckfläche 4 ist ein flexibles Material, wie z. B. Schaumstoff oder Gummi, oder ein Haken, Clip oder ähnliches vorgesehen. In das flexible Material 5 kann eine Nadel des Fadens eingestochen werden. Anstelle des flexiblen Materials kann auch eine andere Befestigungsvorrichtung, wie z. B. ein Haken, ein Bügel, ein Clip und/oder Klebstoff zur Fixierung eines Fadenendes vorgesehen sein. Damit ist ein Fixierungspunkt für ein Ende des Fadens definiert.

Der Grundkörper 2 ist von einem Hüllkörper 6 umgeben. Der Hüllkörper 6 hat ebenfalls eine zylindrische Form mit einem Innenraum 7, der den Grundkörper 2 sowie den hier noch nicht dargestellten Faden aufnimmt. In Längsrichtung, das heißt in Richtung der Zylinderachsen, hat der Grundkörper 2 vorzugsweise eine größere Erstreckung als der Hüllkörper 6, so dass ein oberes Ende des Grundkörpers 2 samt der Deckfläche 4 freiliegt. Dies verbessert die Übersicht und die Handhabung bei der Knüpfung des Knotens.

Der Hüllkörper 6 hat eine längs verlaufende, das heißt in Richtung der Zylinderachsen verlaufende, Öffnung 8. Die Öffnung 8 kann sich über die gesamte Länge des Hüllkörpers 6 oder über einen Teilbereich der Länge des Hüllkörpers 6 erstrecken. Durch die Öffnung 8 werden später ein oder beide Enden des Fadens herausgeführt. Der Hüllkörper 6 hat einen unteren Bereich 9, mittels dessen der Hüllkörper 6 aufrecht abgestellt werden kann. Es kann vorgesehen sein, dass der untere Bereich 9 vollflächig ausgebildet ist, sodass der Grundkörper 2 auf einer inneren Oberfläche des unteren Bereiches 9 steht. Andererseits kann der untere Bereich 9 ringförmig ausgebildet sein, sodass der Grundkörper 2 in dem Hüllkörper 6 frei beweglich ist.

In Figur 2 ist eine Draufsicht auf die Vorrichtung 1 gezeigt. Wie zu sehen ist, umgibt der Zwischenraum 7 den Grundkörper 2 vollständig, sodass ein Faden vollständig, das heißt über den gesamten Winkelbereich von 360° um den Grundkörper 2 herumgelegt werden kann.

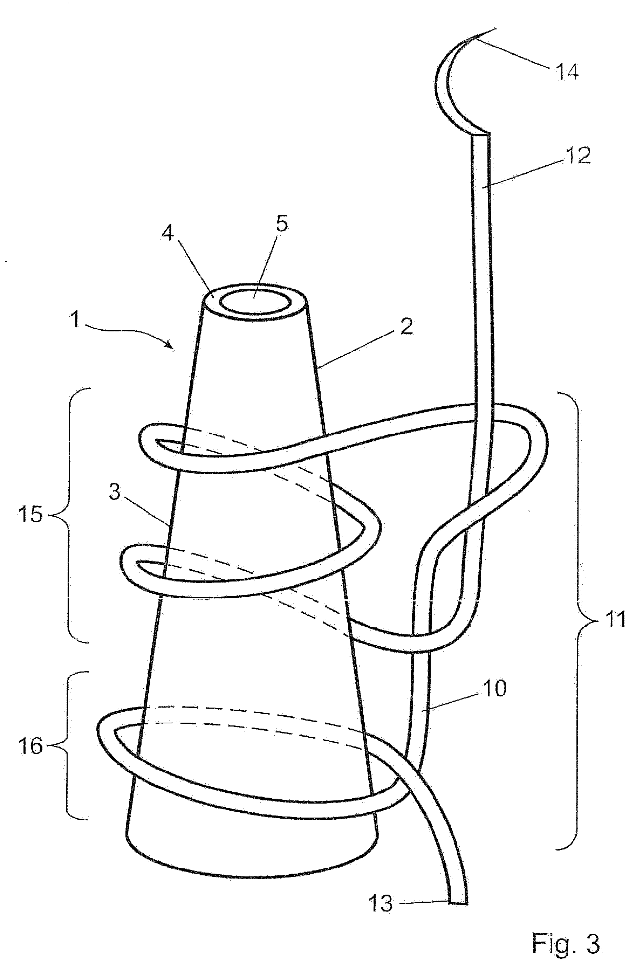

In Figur 3 ist die Vorrichtung 1 mit einem Faden 10 dargestellt. Der Faden 10 ist zu einem vorbereiteten, noch offenen Knoten 11 um die Mantelfläche 3 des Rundkörpers 2 gelegt. Ein erstes oder Funktionsende 12 des Fadens 10 befindet sich im Bereich der Deckfläche 4 und ein zweites oder Zugende 13 befindet sich an einer Basis des Grundkörpers 2. An dem Funktionsende 12 des Fadens 10 ist eine Nadel oder Spitze 14 angeordnet, welche in das flexible Material 5 gestochen werden kann und auf dies Art befestigt werden kann.

Der Faden 10 bzw. der Knoten 11 weist einen ersten Bereich oder Adaptionsknoten 15 sowie einen zweiten Bereich oder Sicherungsknoten 16 auf. Der Adaptionsknoten 15 ist dabei dem Funktionsende 12 bzw. der Deckfläche 4 zugewandt. Der Sicherungsknoten 16 ist dem Zugende 13 bzw. der Grundfläche des Grundkörpers 2 zugewandt. Über den Funktionsknoten 15 werden die an den Knoten 11 gestellten Anforderungen realisiert. Mittels des Sicherungsknotens 16 wird der fertige Knoten dann gegen Auflösen gesichert.

Zur Vorbereitung des Knotens wird der Faden 10 in einem vorbereiteten Knoten 11 um den konus- oder kegelförmigen Grundkörper gewickelt. So wird der Faden 10 samt vorbereitetem Knoten 11 bis zu tatsächlichen Verwendung sicher an dem Grundkörper 2 gehalten. Dazu kann der Faden 10 enger als dargestellt an der Mantelfläche 3 des Grundkörpers 2 anliegen. Zusätzlich kann der in Figur 1 und 2 dargestellte Hüllkörper 6 den Faden 10 und den vorbereiteten Knoten 11 sichern.

Bei einer Verwendung der Vorrichtung 1 zum Knüpfen eines Knotens bzw. bei einem Knüpfen eines Knotens wird zunächst ein Gegenstand mit dem Funktionsende 12 bzw. der Spitze 14 umführt und/oder umstochen. Danach erfolgt die Fixierung der Spitze 14 und damit des Funktionsendes 12 an oder in dem flexiblen Material 5 bzw. einer anderen Befestigungsvorrichtung. Durch Ziehen an dem Zugende 13 in einer Richtung weg von dem Gegenstand und ggf. Bewegung der Vorrichtung 1 und damit des Funktionsendes 12 in der gleichen Richtung erfolgt zunächst eine Umstülpen des vorbereiteten Knotens 11 bzw. der Fadenwicklungen über das Funktionsende 12 hinweg und dann ein Zuziehen des Knotens. Die Anordnung des Fadens 10 auf der Mantelfläche 3 gewährleistet ein sicheres Gleiten des Fadens 10 und die automatische Sicherung des Knotens. Nach Erstellung des Knotens können die beiden Fadenenden mit einer Schere oder einem ähnlichen Gegenstand abgetrennt werden.

Die Vorrichtung 1 kann vorteilhaft insbesondere dort eingesetzt werden, wo mehrfach aufeinanderfolgende Knoten benötigt werden, Zeitdruck herrscht und/oder beengte Platzverhältnisse vorherrschen. So kann die Vorrichtung 1 z. B. für eine Ligatur bzw. Fixation von Gewebestrukturen verwendet werden. Das dabei erforderliche mehrfache aufeinanderfolgende Knoten des Fadens ist mit Hilfe der Vorrichtung 1 bereits vorbereitet, was eine erhebliche Zeitersparnis bedeutet. Wenn Gewebestrukturen unter Spannung stehen, kann es darüber hinaus im herkömmlichen Sinne nach dem Knüpfen eines ersten Knotens zu einer Lockerung dieses Knotens kommen, was durch die Vorrichtung 1 erfolgreich verhindert wird. Insbesondere in der minimalinvasiven Chirurgie (MIC) stellt das Knoten im Körper des Patienten aufgrund des beschränkten Raumes, der zweidimensionalen Visualität und der erforderlichen technischen Versiertheit des Chirurgen eine große Hürde dar. Hier kann die Vorrichtung 1 zur Vernähung oder Ligatur bei minimalinvasiven oder auch offenen Operationen, wie z. B. Darmperforationen, Peritoneum, Gefäßen, Anastomosierungen, Fixierungen, Blutungsstillungen, Mesoskelettierungen, Hautnähten, Gelenken, Fremdkörperstabilisierungen usw. angewandt werden. Von großem Vorteil ist, dass die Armierung oder Bestückung der Vorrichtung 1 mit monofilen oder geflochtenen, resorbierbaren oder nicht resorbierbaren Fäden in vielfältiger Stärke erfolgen kann.

Durch die automatische Sicherung der Ligatur mittels des Sicherungsknotens 16 wird kein zusätzlicher Stabilisationsknoten benötigt. Somit wird der Operationsaufwand vereinfacht und eine Operation zeitverkürzt. Durch die Vorrichtung 1 ist eine sichere Knotung auch von nicht chirurgisch ausgebildeten Personen einfach durchführbar.

Der in Figur 3 vorbereitete Knoten 11 umfasst in dem Adaptionsknotenbereich 15 zwei Schleifen um den Grundkörper 2. Anders ausgedrückt ist der Faden 10 zweimal in einer ersten Richtung um den Grundkörper 2 gelegt oder gewickelt. Bezogen auf das Zugende 13 ist der Faden 10 gegen den Uhrzeigersinn gewickelt und bezogen auf das Funktionsende 12 ist der Faden 10 mit dem Uhrzeigersinn gewickelt. Weiterhin ist der Faden in dem Adaptionsknotenbereich 15 einmal um das Funktionsende 12 bzw. einen dem Funktionsende 12 benachbarten Fadenbereich gewickelt. Diese Wicklung ist in einer zweiten, der ersten Richtung entgegengesetzten Richtung, erfolgt. Zur Bildung eines Sicherungsknotens ist der Faden 10 in der zweiten Richtung einmal um den Grundkörper 2 gelegt. Dies ist im unteren Teil des Grundkörpers 2 angeordnet, das heißt in räumlicher Nähe zu dem Zugende 13.

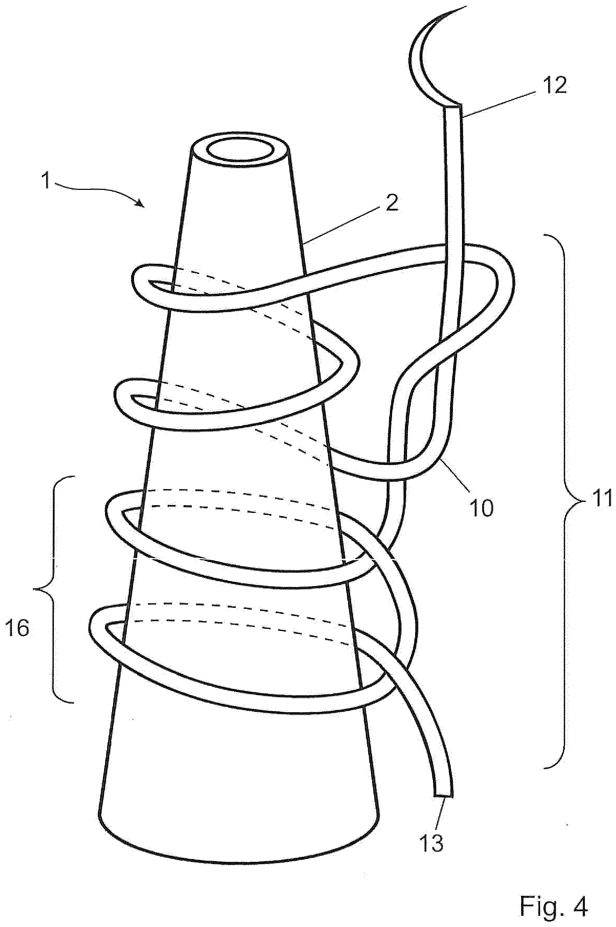

In Figur 4 ist die Vorrichtung 1 mit einem weiteren vorbereiteten Knoten 11 dargestellt. Der Knoten 11 entspricht weitestgehend dem Knoten 11 aus Figur 3. Hier in Figur 4 ist zur Bildung des Sicherungsknotens 16 der Faden 10 in der zweiten Richtung zweimal um den Grundkörper 2 gelegt.

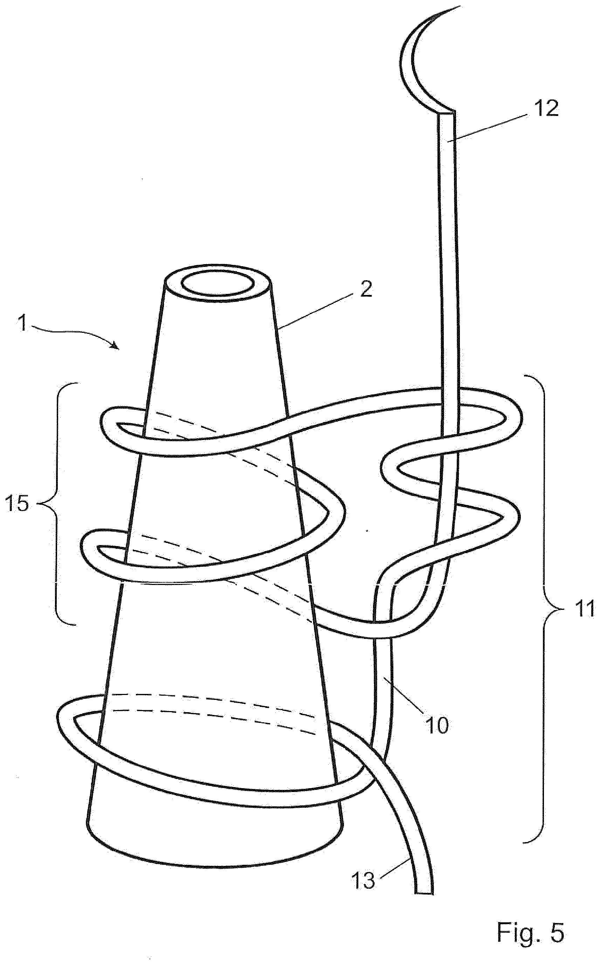

In Figur 5 ist die Vorrichtung 1 mit einem weiteren vorbereiteten Knoten 11 dargestellt. Der Knoten 11 entspricht wiederum im Wesentlichen dem in Figur 3 dargestellten Knoten 11. Im Unterschied zu Figur 3 ist hier bei dem Knoten 11 der Faden 10 zweimal in der zweiten Richtung um das Funktionsende gelegt. Es ist auch möglich, die beiden Knoten aus den Figuren 4 und 5 miteinander zu kombinieren, so dass ein Knoten beispielsweise einen Faden aufweist, der in der zweiten Richtung zweimal um das Funktionsende und in der zweiten Richtung zur Bildung eines Sicherungsknotens zweimal um den Grundkörper 2 gelegt ist.

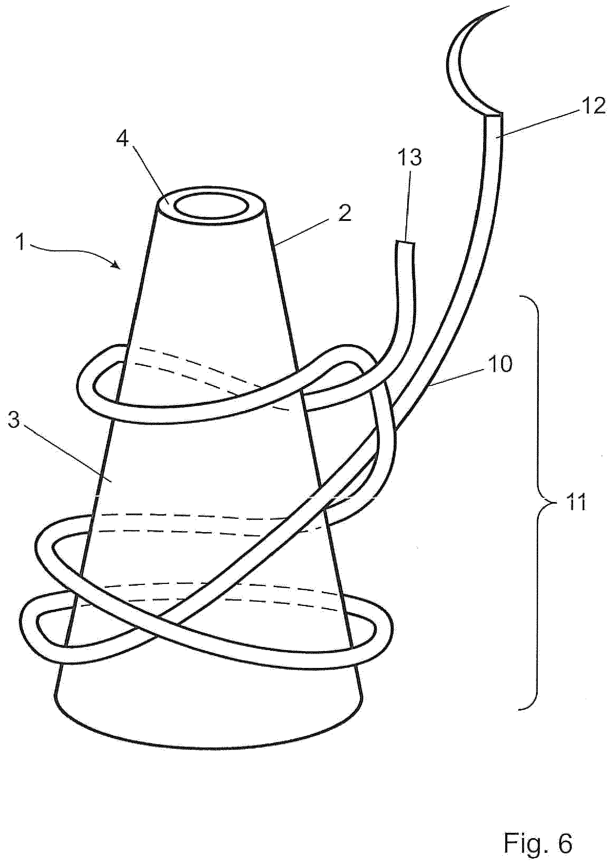

In Figur 6 ist die Vorrichtung 1 mit einem weiteren Knoten 11 dargestellt. Bei diesem Knoten 11 befindet sich zusätzlich zu dem Funktionsende 12 auch das Zugende 13 im Bereich der Kegelspitze oder der Deckfläche 4 des Grundkörpers 2. Während bei den Darstellungen der Figuren 3 bis 5 Überkreuzungen oder Verschlingungen des Fadens 10 neben der Mantelfläche 3 des Grundkörpers 2 angeordnet sind, sind hier in Figur 6 die Überkreuzungen des Fadens 10 im Bereich der Mantelfläche 3 angeordnet. Dies kann unter Umständen die Handhabung der Vorrichtung 1 sowie die Haltbarkeit des vorbereiteten Knotens 11 verbessern.

Die im Folgenden beschriebenen Knoten sind weitere Ausgestaltungen der zuvor beschriebenen Knoten. Diese Knoten können für bestimmte Anwendungen vorteilig sein. So können zum Beispiel zusätzliche Windungen die Dicke des Knotens vergrößern, so dass dieser stabiler ist und/oder nicht durch eine bestimmte Öffnung gleiten kann. Für das Zuziehen der Knoten gilt grundsätzlich, dass das Zugende 13 zum Gegenstand hin gezogen wird und dass das Funktionsende 12 vom Gegenstand weg gezogen wird.

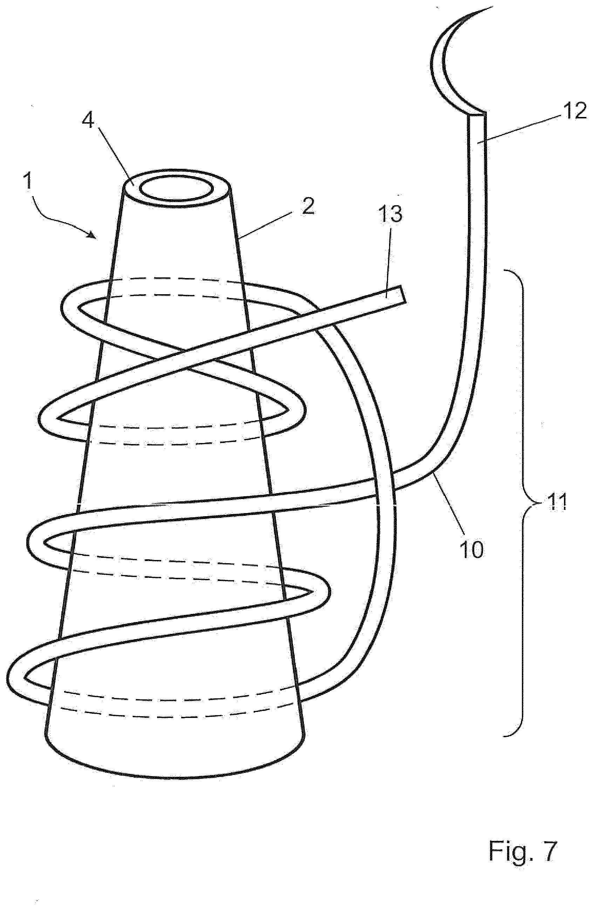

In Figur 7 ist die Vorrichtung 1 mit einem weiteren Knoten 11 dargestellt. Auch bei diesem Knoten befinden sich sowohl das Funktionsende 12 als auch das Zugende 13 im Bereich der Deckfläche 4 des Grundkörpers 2.

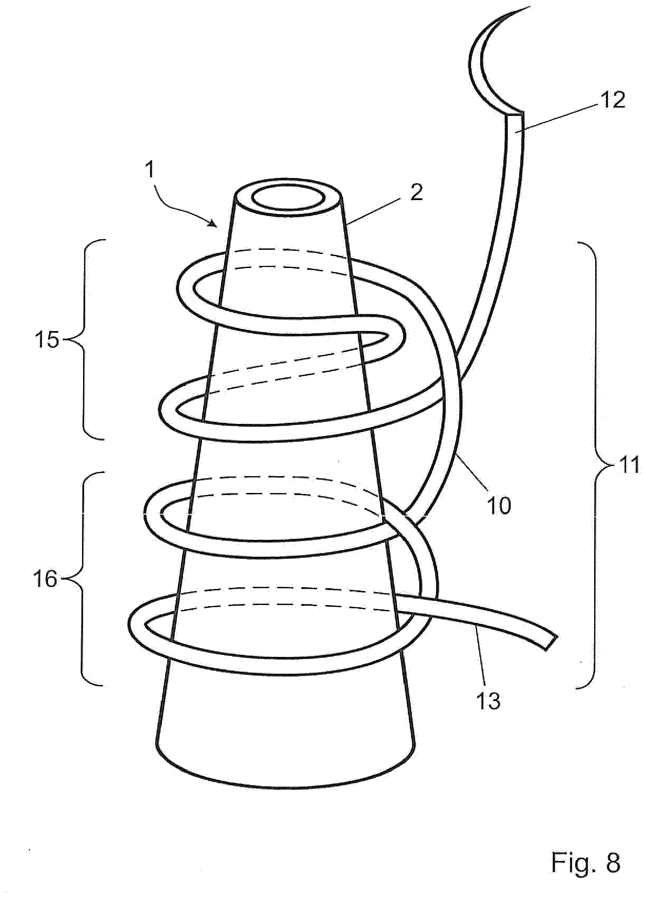

In Figur 8 ist die Vorrichtung 1 mit einem noch weiteren Knoten 11 dargestellt. Dieser Knoten 11 hat wiederum einen Adaptionsknoten 15 in einem oberen Bereich des Grundkörpers 2 und einen Sicherungsknoten, der doppelt ausgeführt ist, in einem unteren Bereich des Grundkörpers 2. In dem Bereich des Sicherungsknotens 16 ist das Zugende 13 des Fadens 10 zugänglich, während ausgehend von dem Funktionsknoten 15 das Funktionsende 12 zugänglich ist.

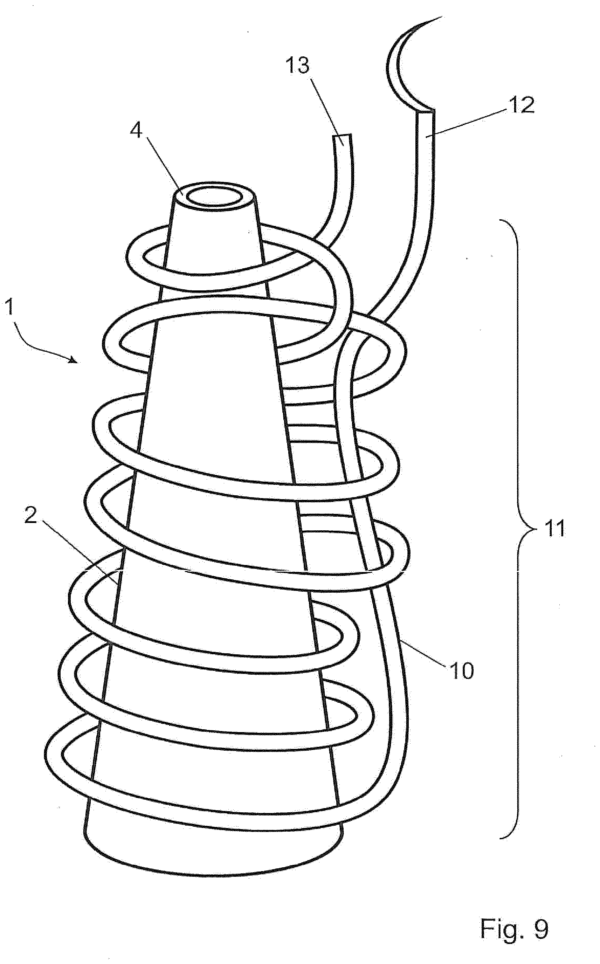

In Figur 9 ist die Vorrichtung 1 mit einem weiteren Knoten 11 dargestellt. Bei diesem Knoten 11 sind wiederum sowohl das Funktionsende 12 als auch das Zugende 13 im Bereich der Deckfläche 4 des Grundkörpers 2 angeordnet. Der Faden 10 ist in einer Vielzahl von Schlingen um den Grundkörper 2 gelegt. Insgesamt ist der Grundkörper 2 sieben Mal mit dem Faden 10 umwickelt, wobei das Funktionsende 12 durch drei dieser Schlingen hindurchgeführt ist.

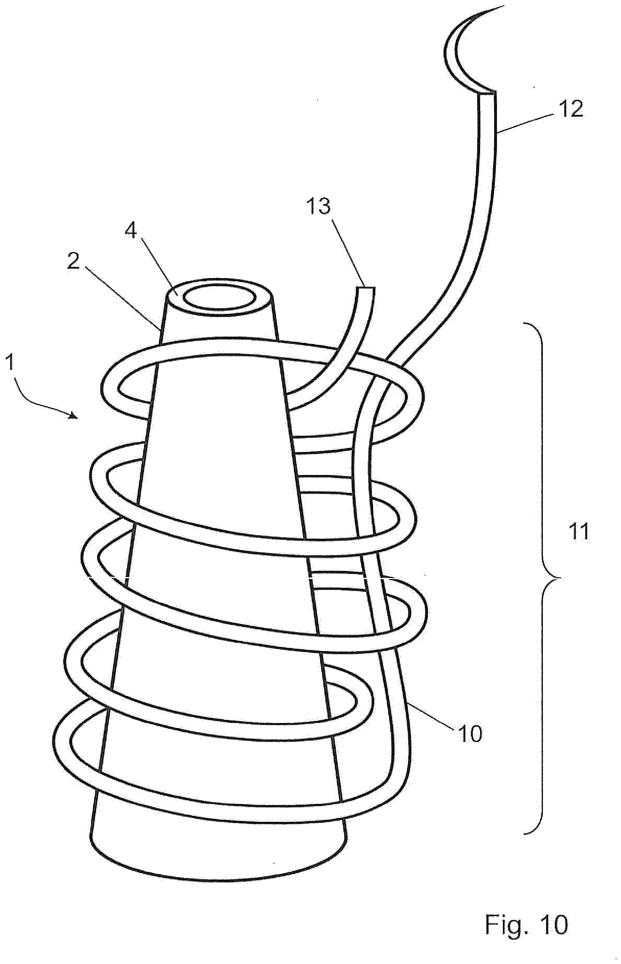

In Figur 10 ist die Vorrichtung 1 mit einem weiteren Knoten 11 dargestellt. Auch bei diesem Knoten 11 befinden sich das Funktionsende 12 und das Zugende 13 im Bereich der Deckfläche 4 des Grundkörpers 2. Hier ist der Faden 10 fünfmal um den Grundkörper 2 geschlungen oder gewickelt, wobei das Funktionsende 12 durch drei der damit entstehenden Windungen hindurchgeführt ist.

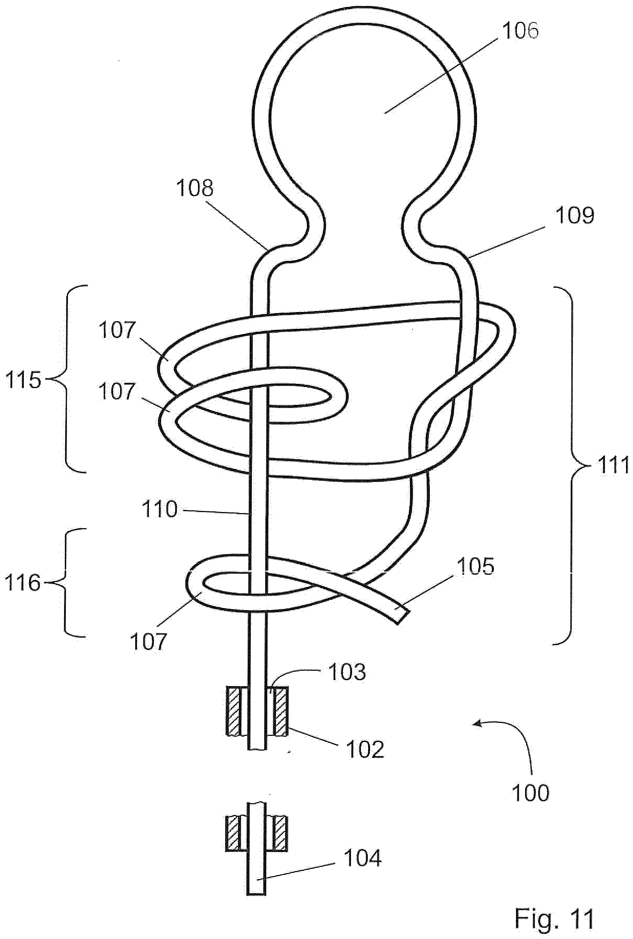

Anhand der Figuren 11 bis 13 wird eine weitere Vorrichtung 100 zum Knüpfen eines Knotens 111 mit einem Faden 110 beschrieben. Die Vorrichtung 100 kann vorteilhaft wie folgt ausgebildet sein:
1. Vorrichtung zum Knüpfen eines Knotens mit einem Faden (110) aufweisend ein Funktionsende (105) und ein Zugende (104), dadurch gekennzeichnet, dass ein hohlzylindrischer Grundkörper (102) vorgesehen ist, durch dessen Hohlraum (103) ein Teil des Fadens (110) verläuft, wobei auf einer Seite des Grundkörpers (102) das Zugende (104) angeordnet ist und wobei auf der anderen Seite der Faden (110) in Form eines vorbereiteten, noch offenen Knotens (111) mit einer Schlinge (106) gelegt ist, so dass durch Ziehen an dem Zugende (104) von der Schlinge (106) weg und an dem Funktionsende (105) zu der Schlinge (106) hin der Faden (110) einen geschlossenen Knoten um einen in der Schlinge (106) anordnenbaren Gegenstand bildet.
2. Vorrichtung nach Punkt 1, dadurch gekennzeichnet, dass der Durchmesser des Hohlraums (103) dem Querschnitt des Fadens (110) entspricht.
3. Vorrichtung nach Punkt 1 oder 2, dadurch gekennzeichnet, dass der Faden (110) einen der Schlinge (106) zugewandten Adaptionsknoten (115) und einen dem Grundkörper (102) zugewandten Sicherungsknoten (116) aufweist.
4. Vorrichtung nach einem der vorhergehenden Punkte, dadurch gekennzeichnet, dass der Faden (110) in einer ersten Richtung mindestens zweimal um einen ersten Teil (108) der Schlinge (106) gelegt ist, der in Richtung des Grundkörpers (102) verläuft, und dass der Faden (110) in einer zweiten Richtung mindestens einmal um den zweiten Teil (109) der Schlinge (106) gelegt ist.
5. Vorrichtung nach einem der vorhergehenden Punkte, dadurch gekennzeichnet, dass der Faden (110) zur Bildung eines Adaptionsknotens (115) in einer ersten Richtung mindestens zweimal um einen ersten Teil (108) der Schlinge (106) gelegt ist, der in Richtung des Grundkörpers (102) verläuft, und dass der Faden (110) in einer zweiten Richtung mindestens einmal um den zweiten Teil (109) der Schlinge (106) gelegt ist und dass der Faden (110) zur Bildung eines Sicherungsknotens (116) in der zweiten Richtung mindestens einmal um den ersten Teil (108) der Schlinge (106) gelegt ist.
6. Verfahren (welches nicht ein Teil der Erfindung ist) zum Knüpfen eines Knotens mit einem Faden (110) aufweisend ein Funktionsende (105) und ein Zugende (104), gekennzeichnet durch die Schritte:
   - Bereitstellen eines hohlzylindrischen Grundkörpers (102), durch dessen Hohlraum (103) ein Teil des Fadens (110) verläuft, wobei auf einer Seite des Grundkörpers (102) das Zugende (104) angeordnet ist und wobei auf der anderen Seite der Faden (110) in Form eines vorbereiteten, noch offenen Knotens (111) mit einer Schlinge (106) gelegt ist;
   - Anordnen eines Gegenstands in der Schlinge (106); und
   - Ziehen an dem Zugende (104) und dem Funktionsende (105), so dass der Faden (110) um den Gegenstand einen geschlossenen Knoten bildet.

In Figur 11 ist die Vorrichtung 100 zum Knüpfen eines Knotens 111 aus oder mit einem Faden 110 dargestellt. Die Vorrichtung 100 umfasst einen hohlzylindrischen Grundkörper 102, beispielsweise in Form eines Hohlrohres, mit einem Innenraum 103. Durch den Innenraum 103 verläuft ein Teil des Fadens 110. Ein Zugende 104 des Fadens 110 ragt auf einer ersten Seite aus dem Grundkörper 102 heraus. Somit befindet sich das Zugende 104 auf einer ersten Seite des Grundkörpers 102. Auf der gegenüberliegenden anderen oder zweiten Seite des Grundkörpers 102 ist der vorbereitete Knoten 111 angeordnet. Aus dem vorbereiteten Knoten 111 ragt ein weiteres Ende oder Funktionsende 105 des Fadens 110 heraus. Das Funktionsende 105 weist in Richtung des Grundkörpers 102. An dem anderen gegenüberliegenden Ende des vorbereiteten Knotens 111, das heißt an dem von dem Grundkörper 102 abgewandten Ende, ist eine Schlinge 106 vorgesehen. Der vorbereitete Knoten 111 ist derart gelegt oder vorbereitet, dass der Faden 110 die offene Schlinge 106 bildet. Die Schlinge kann beispielsweise um einen Gegenstand gelegt werden, an dem der Faden 110 befestigt werden soll oder der beispielsweise durch den Faden zusammen oder abgeschnürt werden soll.

Der in Figur 11 dargestellte vorbereitete Knoten 111 entspricht dem in Figur 3 dargestellten Knoten. Hier in Figur 11 ist der Knoten 111 gewissermaßen um einen weiteren Schritt vorbereitet als der Knoten 11 aus Figur 3. Im Vergleich zu Figur 3 ist das Zugende 104 bereits durch die Schlaufen oder Schlingen 107 hindurchgezogen. In Figur 3 sind diese Schlaufen auf dem Grundkörper2 angeordnet, während das Funktionsende 12, welches dem Zugende 104 aus Figur 11 entspricht noch frei ist.

Entsprechend kann der Knoten 111 wiederum in einem Adaptionsknoten 115 und einen Sicherungsknoten 116 unterteilt werden. Zum besseren Verständnis kann die Schlinge 106 in einem ersten Teil 108, der hier links dargestellt ist, und einen zweiten Teil 109, der hier rechts dargestellt ist, unterteilt werden. Zur Bildung des Adaptionsknotens 115 ist der Faden 110 bzw. die beiden oberen Schlaufen 107 in einer ersten Richtung zweimal um den ersten Teil 108 der Schlinge 106 gelegt. Weiterhin ist der Faden 110 in einer zweiten Richtung um den zweiten Teil 109 der Schlinge 106 gelegt. Zur Bildung des Sicherungsknotens 116 ist der Faden 110 in der zweiten Richtung einmal um den ersten Teil 108 der Schlinge 106 gelegt. Der erste Teil 108 der Schlinge 106 verläuft zu dem Grundkörper 102, während der zweite Teil 109 der Schlinge 106 zu dem Funktionsende 105 verläuft.

Sämtliche in den Figuren 3 bis 10 in Zusammenhang mit dem Grundkörper 2 dargestellten Knoten 11 können entsprechend mit der Vorrichtung 100 verwendet werden. Dazu wird das Funktionsende 12 der Figuren 3 bis 10 jeweils durch die Schlingen geführt, welche auf der Mantelfläche 3 des Grundkörpers 2 aufliegen. Gewissermaßen wird also der Grundkörper 2 durch das Funktionsende 12 ersetzt, sodass aus den vorbereiteten Knoten 11 der Figuren 3 bis 10 ein vorbereiteter Knoten 111 für die Vorrichtung 100 wird.

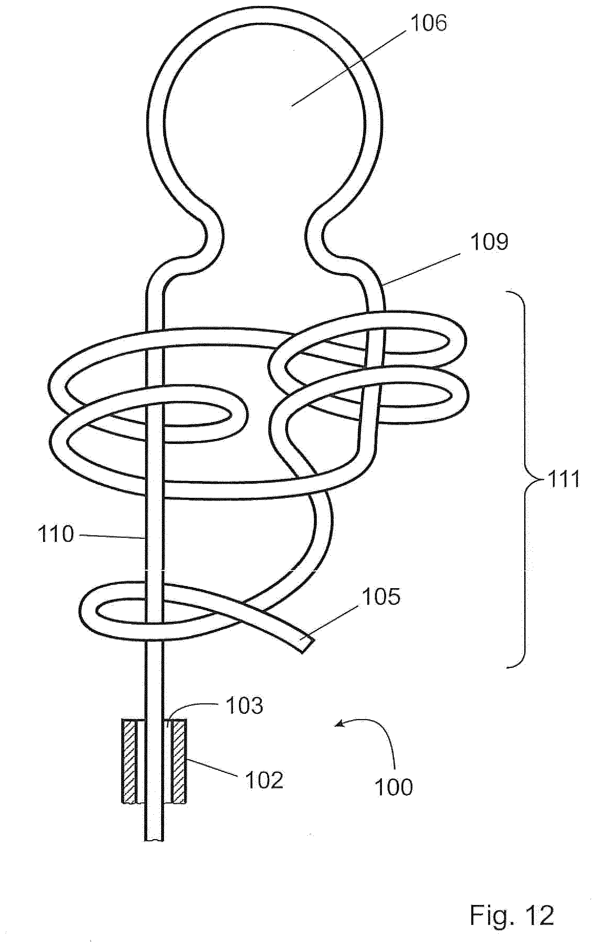

In Figur 12 ist die Vorrichtung 100 mit einem weiteren vorbereiteten Knoten 111 dargestellt. Der Knoten 111 entspricht im Wesentlichen dem in Figur 11 dargestellten Knoten 111. Im Unterschied zu Figur 11 ist hier bei dem Knoten 111 der Faden 110 zweimal in der zweiten Richtung um den zweiten Teil 109 der Schiene 106 gelegt. Der in Figur 12 dargestellte Knoten 111 entspricht dem in Figur 5 dargestellten Knoten 11.

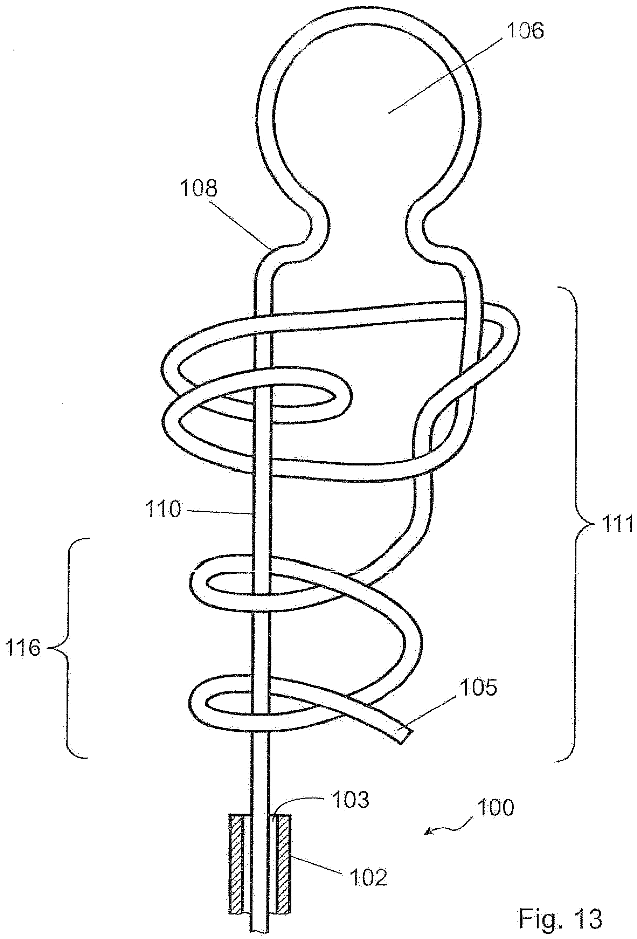

In Figur 13 ist die Vorrichtung 100 mit einem weiteren vorbereiteten Knoten 111 dargestellt. Der Knoten 111 entspricht weitestgehend dem Knoten 111 aus Figur 11. Hier in Figur 13 ist zur Bildung des Sicherungsknotens 116 der Faden 110 in der zweiten Richtung zweimal um den zweiten Teil 108 der Schleife 106 gelegt. Der in Figur 13 dargestellte Knoten 111 entspricht dem in Figur 4 dargestellten Knoten 11.

Die Vorrichtung 100 kann verwendet werden, um Gegenstände mit der Schlinge 106 zu umschlingen und anschließend den vorbereiteten Knoten 111 zuzuziehen, wodurch der Gegenstand und der Faden 110 miteinander verbunden werden. Der Grundkörper 102 erlaubt das Durchführen oder Herausführen des Zugendes 104, um so das Zuziehen des Knotens zu vereinfachen. Der vorbereitete Knoten 111 mit der Schlinge 106 und dem Funktionsende 105 kann dann in oder hinter einem Objekt oder Hindernis angeordnet sein. Der Grundkörper 102 überbrückt oder durchdingt das Objekt, so dass das Zugende 104 sich außerhalb des Objektes befindet und die Schlinge 106 und das Funktionsende 105 sich in oder hinter dem Objekt befinden.

Neben technischen Anwendungen eignet sich die Vorrichtung 100 auch für medizinische Zwecke. Die Vorrichtung 100 kann beispielsweise zum Anschlingen von Gewebestrukturen mit einer Selbstsicherungsfunktion verwendet werden. Der vorgefertigte oder vorbereitete Knoten 111 mit der Schiene 106 kann beispielsweise über eine Trokarhülse intraabdominell eingeführt werden. Die zur Ligatur vorgesehene Gewebestruktur wird durch die Schlinge 106 gezogen. Durch gleichzeitigen Zug des Zugendes 104 von der Schlinge 106 weg und Zug des Funktionsendes 105 zur Schlinge 106 erfolgt das Zuziehen der Schlinge 106 bzw. des Knotens 111. Die beschriebene Anordnung der unterschiedlichen Knoten 111 und des Hohlzylinders 102 gewährleistet ein sicheres Gleiten und die automatische Stabilisierung der Ligatur. Nach Zuziehen der Schlinge 106 bzw. Bildung des Knotens 111 wird das Funktionsende 105 des Fadens 110 intraabdominell in der Nähe der Ligatur mit einer Schere durchtrennt.

Durch die automatische Sicherung der Ligatur wird kein zusätzlicher Stabilisationsknoten benötigt. Somit wird der Aufwand vereinfacht und die Operationszeit verkürzt. Besonders bei Strukturen unter Spannung kann es bei den vorhandenen Schlingensystemen oder beim extrakorporalen Knoten zu einer Lockerung des Knotens kommen. Die erfindungsgemäße Vorrichtung 100 beseitigt diese Nachteile.

Die Vorrichtung 100 kann bei der Ligatur z. B. der Appendix vermiformis (Blinddarm), am Ductus cysticus (Gallenblasengang), bei Gefäßstümpfen, Bergung von Gewebepräparaten etc. während laparoskopischer Operationen bzw. in der offenen Chirurgie bei schwierigen und undurchsichtigen Verhältnissen des OP-Gebietes angewandt werden. Von hohem Vorteil ist, dass die Armierung des Apparates mit monofilem/geflochtenem und resorbierbaren/nicht resorbierbaren Faden in vielfältiger Stärke stattfinden kann.

## Patentansprüche

1. Vorrichtung zum Knüpfen eines Knotens, wobei die Vorrichtung einen Faden (10) umfasst aufweisend ein Funktionsende (12) und ein Zugende (13), wobei ein Grundkörper (2) vorgesehen ist, um dessen Außenfläche (3) der Faden (10) in Form eines vorbereiteten, noch offenen Knotens (11) gelegt ist, so dass durch Ziehen an dem Zugende (13) der Faden an der Außenfläche (3) entlanggleitend einen geschlossenen Knoten bildet, und wobei der vorbereitete, noch offene Knoten (11) einen dem Funktionsende (12) zugewandten Adaptionsknoten (15) und einen dem Zugende (13) zugewandten Sicherungsknoten (16) aufweist, wobei der Faden (10) zur Bildung des Adaptionsknotens (15) in einer ersten Umwicklungsrichtung mindestens zweimal um den Grundkörper (2) und in einer zweiten, der ersten Umwicklungsrichtung mindestens einmal um das Funktionsende (12) gelegt ist und wobei der Faden (10) zur Bildung des Sicherungsknotens (16) in der zweiten Umwicklungsrichtung mindestens einmal um den Grundkörper (2) gelegt ist, wobei an dem Funktionsende (12) des Fadens (10) eine Nadel oder Spitze angeordnet ist, **dadurch gekennzeichnet, dass** die erste Umwicklungsrichtung der zweiten Umwicklungsrichtung entgegengesetzt ist.

2. Vorrichtung nach Anspruch 1, **dadurch gekennzeichnet, dass** das Funktionsende (12) an einem Fixierungspunkt des Grundkörpers (2) befestigbar ist.

3. Vorrichtung nach Anspruch 2, **dadurch gekennzeichnet, dass** der Fixierungspunkt an einer oberen oder unteren Deckfläche (4) des Grundkörpers (2), an einer Innenfläche des Grundkörpers (2) oder an der Außenfläche (3) angeordnet ist.

4. Vorrichtung nach einem der Ansprüche 1 bis 3, **dadurch gekennzeichnet, dass** an dem Faden (10) vor dem Funktionsende (12) Befestigungsmittel zur Verbindung mit einem Gegenstand und/oder Funktionsmittel zur funktionalen Verbindung mit einem Gegenstand angeordnet sind.

5. Vorrichtung nach Anspruch 4, **dadurch gekennzeichnet, dass** das Befestigungsmittel und/oder das Funktionsmittel eine Nadel, einen Anker und/oder eine künstliche Sehne umfasst.

6. Vorrichtung nach einem der Ansprüche 2 bis 5, **dadurch gekennzeichnet, dass** an dem Fixierungspunkt flexibles Material angeordnet ist.

7. Vorrichtung nach einem der Ansprüche 2 bis 6, **dadurch gekennzeichnet, dass** der Fixierungspunkt und das Funktionsende (12) mittels einer Rastverbindung, einer Bajonettverbindung, einer Klebverbindung oder einer Schließverbindung verbindbar sind, wobei die Verbindung unlösbar oder wieder verschließbar ist.

8. Vorrichtung nach einem der vorhergehenden Ansprüche, **dadurch gekennzeichnet, dass** zusätzlich ein den Grundkörper (2) und Faden (10) zumindest teilweise umgebender Hüllkörper (6) vorgesehen ist, wobei der Hüllkörper (6) mindestens eine im Bereich der Mantelfläche (3) angeordnete Öffnung (8) für das Funktionsende (12) und/oder das Zugende (13) aufweist.

9. Vorrichtung nach einem der vorhergehenden Ansprüche, **dadurch gekennzeichnet, dass** der Grundkörper (2) zylinderförmig, kegelstumpfförmig, quaderförmig, prismatisch oder speichenförmig mit radialen Ausläufern ausgebildet ist.

10. Vorrichtung nach einem der vorhergehenden Ansprüche, **dadurch gekennzeichnet, dass** an dem Funktionsende (12) ein Element zur Befestigung an einem Gegenstand angeordnet ist.

## Claims

1. A device for tying a knot, said device comprising a thread (10) having a functional end (12) and a pulling end (13), wherein a base body (2) is provided around whose outer surface (3) the thread (10) is laid in the form of a prepared, still open knot (11), so that by pulling on the pulling end (13) the thread forms a closed knot sliding along the outer surface (3), and wherein the prepared, still open knot (11) has an adaptation knot (15) facing the functional end (12) and a securing knot (16) facing the pulling end (13), wherein the thread (10) for forming the adaptation knot (15) is wrapped at least twice around the base body (2) in a first wrapping direction and at least once around the functional end (12) in a second wrapping direction different from the first wrapping direction and wherein the thread (10) for forming the securing knot (16) is wrapped at least once around the base body (2) in the second wrapping direction, wherein a needle or point is arranged at the functional end (12) of the thread (10), **characterized in that** the first winding direction is opposite to the second winding direction.

2. device according to claim 1, **characterized in that** the function end (12) can be fastened to a fixing point of the base body (2).

3. device according to claim 2, **characterized in that** the fixing point is arranged on an upper or lower cover surface (4) of the base body (2), on an inner surface of the base body (2) or on the outer surface (3).

4. device according to any one of claims 1 to 3, **characterized in that** fastening means for connection to an object and/or functional means for functional connection to an object are arranged on the thread (10) before the functional end (12).

5. device according to claim 4, **characterized in that** the fastening means and/or the functional means comprises a needle, an anchor and/or an artificial tendon.

6. device according to any one of claims 2 to 5, **characterized in that** flexible material is arranged at the fixation point.

7. device according to any one of claims 2 to 6, **characterized in that** the fixing point and the functional end (12) can be connected by means of a latching connection, a bayonet connection, an adhesive connection or a closing connection, the connection being non-detachable or reclosable.

8. device according to one of the preceding claims, **characterized in that** additionally a sheathing body (6) at least partially surrounding the base body (2) and thread (10) is provided, the sheathing body (6) having at least one opening (8) arranged in the region of the sheathing surface (3) for the functional end (12) and/or the tension end (13).

9. device according to one of the preceding claims, **characterized in that** the base body (2) is cylindrical, frustoconical, cuboidal, prismatic or spoke-shaped with radial extensions.

10. device according to any one of the preceding claims, **characterized in that** an element for attachment to an object is arranged at the functional end (12).

## Revendications

1. Dispositif pour nouer un noeud, le dispositif comprenant un fil (10) présentant une extrémité fonctionnelle (12) et une extrémité de traction (13), un corps de base (2) étant prévu, autour de la surface extérieure (3) duquel le fil (10) est posé sous la forme d'un noeud (11) préparé, encore ouvert, de sorte qu'en tirant sur l'extrémité de traction (13), le fil forme un noeud fermé en glissant le long de la surface extérieure (3), et dans lequel le noeud (11) préparé et encore ouvert présente un noeud d'adaptation (15) tourné vers l'extrémité fonctionnelle (12) et un noeud de sécurité (16) tourné vers l'extrémité de traction (13), le fil (10) pour former le noeud d'adaptation (15) étant passé au moins deux fois autour du corps de base (2) dans une première direction d'enroulement et au moins une fois autour de l'extrémité fonctionnelle (12) dans une deuxième direction d'enroulement, la première et le fil (10) pour former le noeud de sécurité (16) étant passé au moins une fois autour du corps de base (2) dans la deuxième direction d'enroulement, une aiguille ou une pointe étant disposée à l'extrémité fonctionnelle (12) du fil (10), **caractérisé en ce que** le premier sens d'enroulement est opposé au deuxième sens d'enroulement.

2. dispositif selon la revendication 1, **caractérisé en ce que** l'extrémité fonctionnelle (12) peut être fixée à un point de fixation du corps de base (2).

3. dispositif selon la revendication 2, **caractérisé en ce que** le point de fixation est disposé sur une surface de recouvrement supérieure ou inférieure (4) du corps de base (2), sur une surface intérieure du corps de base (2) ou sur la surface extérieure (3).

4. dispositif selon l'une des revendications 1 à 3, **caractérisé en ce que** des moyens de fixation pour la liaison avec un objet et/ou des moyens fonctionnels pour la liaison fonctionnelle avec un objet sont disposés sur le fil (10) avant l'extrémité fonctionnelle (12).

5. dispositif selon la revendication 4, **caractérisé en ce que** le moyen de fixation et/ou le moyen fonctionnel comprend une aiguille, une ancre et/ou une corde artificielle.

6. Dispositif selon l'une quelconque des revendications 2 à 5, **caractérisé en ce qu'**un matériau flexible est disposé au niveau du point de fixation.

7. Dispositif selon l'une des revendications 2 à 6, **caractérisé en ce que** le point de fixation et l'extrémité fonctionnelle (12) peuvent être reliés au moyen d'une liaison par encliquetage, d'une liaison à baïonnette, d'une liaison par collage ou d'une liaison de fermeture, la liaison étant inamovible ou pouvant être refermée.

8. Dispositif selon l'une des revendications précédentes, **caractérisé en ce qu'**il est prévu en outre un corps enveloppant (6) entourant au moins partiellement le corps de base (2) et le fil (10), le corps enveloppant (6) présentant au moins une ouverture (8) disposée dans la zone de la surface d'enveloppe (3) pour l'extrémité fonctionnelle (12) et/ou l'extrémité de traction (13).

9. Dispositif selon l'une des revendications précédentes, **caractérisé en ce que** le corps de base (2) est de forme cylindrique, tronconique, parallélépipédique, prismatique ou en forme de rayon avec des prolongements radiaux.

10. Dispositif selon l'une des revendications précédentes, **caractérisé en ce qu'**un élément de fixation à un objet est disposé à l'extrémité fonctionnelle (12).
